# EUROPEAN PATENT SPECIFICATION

(11) **EP 2 836 220 B1**
(45) Date of publication and mention of the grant of the patent: **30.05.2018**
(21) Application number: 13775009.7
(22) Date of filing: 10.04.2013
(51) Int. Cl.: A61K 31/7088, A61K 35/76, A61P 35/00, C12N 15/86

(54) **METHODS FOR BLADDER CANCER THERAPY USING BACULOVIRAL VECTORS**
VERFAHREN FÜR BLASENKREBSTHERAPIE UNTER VERWENDUNG VON BACULOVIRUSVEKTOREN
MÉTHODES DE TRAITEMENT DU CANCER DE LA VESSIE AU MOYEN DE VECTEURS BACULOVIRAUX

(30) Priority: 10.04.2012 US 201261622376 P
(43) Date of publication of application: 18.02.2015
(73) Proprietor: Agency for Science, Technology and Research, Singapore 138632 (SG)
(72) Inventor: WANG, Shu, Singapore 138669 (SG); WU, Chunxiao, Singapore 138669 (SG); ZHAO, Ying, Singapore 138669 (SG); LEE, Esther, Xingwei, Singapore 138669 (SG)
(74) Representative: Awapatent AB
(86) International application number: PCT/SG2013/000142
(87) International publication number: WO 2013/154503

(56) References cited:
- WO-A2-98/11243
- TANI HIDEKI ET AL: "In vitro and in vivo gene delivery by recombinant baculoviruses", JOURNAL OF VIROLOGY, THE AMERICAN SOCIETY FOR MICROBIOLOGY, US, vol. 77, no. 18, 1 September 2003 (2003-09-01), pages 9799-9808, XP002380522, ISSN: 0022-538X, DOI: 10.1128/JVI.77.18.9799-9808.2003
- HOFMANN C ET AL: "EFFICIENT GENE TRANSFER INTO HUMAN HEPATOCYTES BY BACULOVIRUS VECTORS", PROCEEDINGS OF THE NATIONAL ACADEMY OF SCIENCES, NATIONAL ACADEMY OF SCIENCES, US, vol. 92, 1 October 1995 (1995-10-01), pages 10099-10103, XP002072661, ISSN: 0027-8424, DOI: 10.1073/PNAS.92.22.10099
- BURKE ET AL: "Virus therapy for bladder cancer", CYTOKINE AND GROWTH FACTOR REVIEWS, ELSEVIER LTD, GB, vol. 21, no. 2-3, 1 April 2010 (2010-04-01), pages 99-102, XP027066629, ISSN: 1359-6101, DOI: 10.1016/J.CYTOGFR.2010.02.003 [retrieved on 2010-04-09]
- KAZUHIRO MATSUMOTO ET AL: "Intravesical Interleukin-15 Gene Therapy in an Orthotopic Bladder Cancer Model", HUMAN GENE THERAPY, vol. 22, no. 11, 1 November 2011 (2011-11-01), pages 1423-1432, XP055232150, US ISSN: 1043-0342, DOI: 10.1089/hum.2011.013 -& MATSUMOTO K ET AL: "INTRAVESICAL INTERLEUKIN-15 GENE THERAPY IN A MOUSE ORTHOTOPIC BLADDER CANCER MODEL", JOURNAL OF UROLOGY, LIPPINCOTT WILLIAMS & WILKINS, BALTIMORE, MD, US, vol. 181, no. 4, 1 April 2009 (2009-04-01) , page 349, XP026009042, ISSN: 0022-5347, DOI: 10.1016/S0022-5347(09)60993-9 [retrieved on 2009-03-18]
- KAUFMAN J ET AL: "State of the art in intravesical therapy for lower urinary tract symptoms", REVIEWS IN UROLOGY, MEDREVIEWS, NEW YORK, US, vol. 12, no. 4, 1 October 2010 (2010-10-01), pages E181-E189, XP002623158, ISSN: 1523-6161, DOI: 10.3909/RIU0496
- SHOJI I ET AL: "Efficient transfer ino various mammalian cells, including non-hepatic cells, by baculovirus vectors", JOURNAL OF GENERAL VIROLOGY, SOCIETY FOR GENERAL MICROBIOLOGY, SPENCERS WOOD, GB, vol. 78, no. 10, 1 October 1997 (1997-10-01), pages 2657-2664, XP002100056, ISSN: 0022-1317
- KOST T A ET AL: "Recombinant baculoviruses as mammalian cell gene-delivery vectors", TRENDS IN BIOTECHNOLOGY, ELSEVIER PUBLICATIONS, CAMBRIDGE, GB, vol. 20, no. 4, 1 April 2002 (2002-04-01), pages 173-180, XP004344216, ISSN: 0167-7799, DOI: 10.1016/S0167-7799(01)01911-4
- PIERONI L ET AL: "TOWARDS THE USE OF BACULOVIRUS AS A GENE THERAPY VECTOR", CURRENT OPINION IN MOLECULAR THERAPEUTICS, CURRENT DRUGS, LONDON, GB, vol. 3, no. 5, 1 October 2001 (2001-10-01) , pages 464-467, XP001120522, ISSN: 1464-8431
- GUHASARKAR S ET AL: "Intravesical drug delivery: Challenges, current status, opportunities and novel strategies", JOURNAL OF CONTROLLED RELEASE, ELSEVIER, AMSTERDAM, NL, vol. 148, no. 2, 1 December 2010 (2010-12-01), pages 147-159, XP027509905, ISSN: 0168-3659, DOI: 10.1016/J.JCONREL.2010.08.031 [retrieved on 2010-09-08]
- MAHONEN ET AL: "Post-transcriptional regulatory element boosts baculovirus-mediated gene expression in vertebrate cells", JOURNAL OF BIOTECHNOLOGY, ELSEVIER SCIENCE PUBLISHERS, AMSTERDAM, NL, vol. 131, no. 1, 13 July 2007 (2007-07-13) , pages 1-8, XP022151834, ISSN: 0168-1656, DOI: 10.1016/J.JBIOTEC.2007.05.022
- SIEMENS ET AL: "EVALUATION OF GENE TRANSFER EFFICIENCY BY VIRAL VECTORS TO MURINE BLADDER EPITHELIUM", JOURNAL OF UROLOGY, LIPPINCOTT WILLIAMS & WILKINS, BALTIMORE, MD, US, vol. 165, no. 2, 1 February 2001 (2001-02-01), pages 667-671, XP005545086, ISSN: 0022-5347, DOI: 10.1097/00005392-200102000-00091
- SUH, H.J. ET AL.: 'The Effect of Recombinant Baculovims (BacG-CMV-P53) Mediated Gene Therapy in the HT-1376 Bladder Cancer Cell Line' KOREAN JOURNAL OF UROLOGY vol. 45, 2004, pages 1156 - 1161, XP008175167
- LINDQVIST, C. ET AL.: 'Local AdCD40L Gene Therapy is Effective for Disseminated Murine Experimental Cancer by Breaking T-cell Tolerance and Inducing Tumor Cell Growth Inhibition' JOURNAL OF IMMUNOTHERAPY vol. 32, 2009, pages 785 - 792, XP008145917

### SEQUENCE LISTING

<110> AGENCY FOR SCIENCE, TECHNOLOGY AND RESEARCH WANG, Shu WU, Chunxiao ZHAO, Ying LEE, Esther xingwei
<120> METHODS FOR BLADDER CANCER THERAPY USING BACULOVIRAL VECTORS
<130> 93231-875
<150> US 61/622,376
   <151> 2012-04-10
<160> 3
<170> PatentIn version 3.5
<210> 1
   <211> 583
   <212> DNA
   <213> human cytomegalovirus
<400> 1
   ttaatagtaa tcaattacgg ggtcattagt tcatagccca tatatggagt tccgcgttac       60
   ataacttacg gtaaatggcc cgcctggctg accgcccaac gacccccgcc cattgacgtc      120
   aataatgacg tatgttccca tagtaacgcc aatagggact ttccattgac gtcaatgggt      180
   ggagtattta cggtaaactg cccacttggc agtacatcaa gtgtatcata tgccaagtac      240
   gccccctatt gacgtcaatg acggtaaatg gcccgcctgg cattatgccc agtacatgac      300
   cttatgggac tttcctactt ggcagtacat ctacgtatta gtcatcgcta ttaccatggt      360
   gatgcggttt tggcagtaca tcaatgggcg tggatagcgg tttgactcac ggggatttcc      420
   aagtctccac cccattgacg tcaatgggag tttgttttgg caccaaaatc aacgggactt      480
   tccaaaatgt cgtaacaact ccgccccatt gacgcaaatg ggcggtaggc gtgtacggtg      540
   ggaggtctat ataagcagag ctggtttagt gaaccgtcag atc                        583
<210> 2
   <211> 579
   <212> DNA
   <213> woodchuck hepatitis virus
<400> 2
   cgataatcaa cctctggatt acaaaatttg tgaaagattg actggtattc ttaactatgt       60
   tgctcctttt acgctatgtg gatacgctgc tttaatgcct ttgtatcatg ctattgcttc      120
   ccgtatggct ttcattttct cctccttgta taaatcctgg ttgctgtctc tttatgagga      180
   gttgtggccc gttgtcaggc aacgtggcgt ggtgtgcact gtgtttgctg acgcaacccc      240
   cactggttgg ggcattgcca ccacctgtca gctcctttcc gggactttcg ctttccccct      300
   ccctattgcc acggcggaac tcatcgccgc ctgccttgcc cgctgctgga caggggctcg      360
   gctgttgggc actgacaatt ccgtggtgtt gtcggggaag ctgacgtcct ttccatggct      420
   gctcgcctgt gttgccacct ggattctgcg cgggacgtcc ttctgctacg tcccttcggc      480
   cctcaatcca gcggaccttc cttcccgcgg cctgctgccg gctctgcggc ctcttccgcg      540
   tcttcgcctt cgccctcaga cgagtcggat ctccctttg                             579
<210> 3
   <211> 288
   <212> DNA
   <213> human T-cell leukemia virus type 1
<400> 3
   ggctcgcatc tctccttcac gcgcccgccg ccctacctga ggccgccatc cacgccggtt       60
   gagtcgcgtt ctgccgcctc ccgcctgtgg tgcctcctga actgcgtccg ccgtctaggt      120
   aagtttaaag ctcaggtcga gaccgggcct ttgtccggcg ctcccttgga gcctacctag      180
   actcagccgg ctctccacgc tttgcctgac cctgcttgct caactctacg tctttgtttc      240
   gttttctgtt ctgcgccgtt acagatccaa gctgtgaccg gcgcctac                  288

## Description

### FIELD OF THE INVENTION

The present invention relates to methods for treating or preventing bladder cancer using baculoviral vectors.

### BACKGROUND OF THE INVENTION

Bladder cancer is the most common form of malignancy in the urinary tract. In the United States, 69,000 new cases and close to 15,000 deaths were expected in 2011 (Siegel R et al., 2011). In Singapore, bladder cancer is the 9th most common cancer in men.

Almost all bladder cancers arise from the transitional epithelium and are thus known as transitional cell carcinoma (TCC). The majority of TCC of the bladder are superficial, non-muscle-invasive at initial diagnosis. However, the recurrence rate of superficial TCC after transurethral resection could be as high as 70%, necessitating adjuvant therapy to control recurrence and progression.

Adjuvant therapy is often given in the form of intravesical immunotherapy using Bacillus-Calmette-Cuerin (BCG) for patients with intermediate- or high-risk of cancer recurrence. BCG is produced from attenuated live bovine tuberculosis bacterium and can activate innate immune responses mediated by CD4+ Th1 cytokines, such as interleukin-2 (IL-2), IL-12, IL-18 and interferon-gamma (INF-γ). However, up to 40% of patients will fail BCG immunotherapy within the first year due to BCG refractory, resistance, relapsing, or intolerance (Zlotta AR et al., 2009; Chiong & Esuvaranathan, 2010). Thus, there is a need to develop new adjuvant therapies to improve treatment outcomes for patients with superficial bladder cancer.

Viral vector-based gene therapy that have been explored as a possible adjuvant treatments for bladder cancer include vaccinia virus (Lee SS et al., 1994; Gomella LG et al., 2001; Siemens DR et al., 2003; Fodor I, et al., 2005), adenovirus (Morris BD et al., 1994; Sutton MA et al., 2007; Kuball J et al., 2002; Siemens DR et al., 2003; Pagliaro LC et al., 2003; Malmstrom PU et al., 2010), canarypox virus (Siemens DR et al., 2003), reovirus (Hanel EG et al., 2004), retrovirus (Shiau AL et al, 2001; Kimura T et al., 2003; Dumey N et al, 2005; Kikuchi E et al., 2007), lentivirus (Kikuchi E et al., 2004), and vesicular stomatitis virus (Hadaschik BA et al., 2008). At least 5 clinical trials have been reported on the use of replication-competent vaccinia virus and adenoviral vectors to treat bladder cancer (Gomella LG et al., 2001; Kuball J et al., 2002; Pagliaro LC et al., 2003; Burke J, 2010; Malmstrom PU et al., 2010).

Among the various types of viral vectors tested for cancer gene therapy are retroviruses. The life cycle of the retroviruses includes an integrated state in the host genome, thus allowing a long-term, stable expression of therapeutic genes. One major concern over the use of retroviral vectors is that preferential integration into transcriptionally active regions of genomes by these vectors might lead to insertional mutagenesis, oncogene activation and cellular transformation. The development of leukemia in several children in France and the UK following gene therapy for SCID-X1 (Hacein-Bey-Abina et al., 2003) has brought intense scrutiny to the potential risk associated with the viral vectors.

Viral vectors derived from adenovirus and adeno-associated virus (AAV) have a much lower risk of insertional mutagenesis and have also been tested for cancer gene therapy (Cross & Burmester, 2006; Palmer et al., 2006). Adenoviral vectors are also widely tested for bladder cancer gene therapy in animal models and at least 4 early stage clinical trials (Kuball J et al., 2002; Pagliaro LC et al., 2003; Burke J, 2010; Malmstrom PU et al., 2010). However, as infectious human viruses, adenovirus and AAV activate the human immune system (Calcedo R et al., 2009; Huang & Yang, 2009; Nayak & Herzog, 2010). AAV vectors are efficient in activating B cells (Huang & Yang, 2009) and specific antibodies against AAV2 are detected in 35 to 80% of individuals depending on age group and geographic location (Calcedo R et al., 2009). Although at a very low frequency, AAV capsid-specific CD8+ memory T cells are present in humans and can be reactivated upon AAV transduction (Nayak & Herzog, 2010). Pre-existing immunity against adenovirus is even more prevalent (Bessis N et al., 2004; Nayak & Herzog, 2010). Pre-existing antibodies against group C adenovirus were detected in more than 97% of humans and those against the serotype 2 adenovirus that are commonly used to generate adenoviral vectors in approximately 50% of humans (Nayak & Herzog, 2010). As for T cell-mediated immunity, CD4+ and CD8+ cross-reactive T cells against different adenovirus serotypes were detected in human peripheral blood (Nayak & Herzog, 2010).

Pre-existing immunity is a concern for the use of vectors derived from human infectious viruses in gene therapy in view of possible undesired rejection responses. Under the circumstance that the pre-existing antiviral immunity does not trigger severe pathological changes, it might still be able to inactivate the viral vectors, therefore affecting transduction efficiency. In patients without the pre-existing immunity, adenoviral vectors will rapidly elicit strong antiviral immunity following the first administration since this virus is highly immunogenic. This reaction will preclude further use of the vectors or make subsequent use less effective.

### SUMMARY OF THE INVENTION

Viruses, either replication-competent viruses or replication-deficient recombinant viral vectors, have been tested for bladder cancer therapy in tumor models. However, human infectious viruses as bladder therapy have significant drawbacks, including problems associated with pre-existing immunity and strong immunogenicity. In contrast, the inventors have found that insect baculovirus-based vectors may provide a suitable vehicle for bladder cancer therapy.

Thus, the methods described herein relate to use of baculovirus to treat bladder cancer, including as an adjuvant therapy to prevent or reduce risk of recurrence of bladder cancer following resection of a superficial tumor. Given the limitations in effectiveness and safety of current viral vector systems, baculovirus vectors provide a viable alternative, for use as an adjuvant therapy to induce immunity or as a vector for delivery of a therapeutic nucleic acid.

The inventors have demonstrated that intravesically delivered baculoviral vectors can transduce a normal (non-cancer bearing) mouse bladder effectively. A baculoviral vector that incorporates a mammalian expression cassette containing the human cytomegalovirus immediate-early gene promoter, the woodchuck hepatitis virus post-transcriptional regulatory elements, and the R segment and part of the U5 sequence of long terminal repeat from the human T-cell leukemia virus type 1 into the viral genome may provide high levels of *in vivo* transduction efficiency.

As well, the inventors have found that baculoviral transduction alone, without any therapeutic transgene included in the baculoviral vector, can stimulate antitumor immunity. Using murine cytokine/chemokine antibody arrays to analyze bladder tissue extracts collected from mice receiving intravesical administration of baculoviruses without any transgenes, 59% of the proteins in the array showed a greater than 2-fold increase in expression, including up-regulatation of granulocyte-macrophage colony-stimulating factor, granulocyte colony-stimulating factor, and cutaneous T cell-attracting chemokine. Treatment with baculovirus was found to prolong survival of orthotopic bladder cancer-bearing mice, with 50% of the animals surviving beyond six months.

Using baculoviral vectors for intravesical delivery of the CD40 ligand gene into the bladder of mice with aggressive orthotopic bladder tumor progression, preferential transduction of the tumor followed by reduction of tumor growth and average bladder weight was observed.

Thus, direct intravesical instillation of baculovirus and baculoviral gene transfer vectors may provide a novel therapeutic modality for bladder cancer.

The cell may be a bladder cancer cell or a healthy, non-cancer bladder cell, and may be a cell *in vitro* or *in vivo,* including in a subject in need of treatment of bladder cancer. When the cell is a cell in a subject, contacting may comprise administering the baculoviral vector to the subject by intravesical instillation.

The baculoviral vector further comprises a transgene for expression in the bladder cell operably linked to a promoter that drives expression of the transgene in the bladder cell. For example, the transgene may be a therapeutic transgene for treating bladder cancer, including for example a therapeutic transgene encoding CD40L or IL-15.

In the baculoviral vector, the promoter may comprise the human cytomegalovirus immediate early promoter, including comprising the sequence set forth in SEQ ID NO: 1.

The baculoviral vector may further comprise post-transcriptional regulatory elements from the woodchuck hepatitis virus, in the 3' untranslated region of the transgene, including for example comprising the sequence set forth in SEQ ID NO: 2.

The baculoviral vector may further comprise the R segment and at least a portion of the U5 sequence of the long terminal repeat from the human T-cell leukemia virus type 1, in the 5' untranslated region of the transgene, including for example comprising the sequence set forth in SEQ ID NO: 3.

The method may further comprise adding a transfection agent either prior to or concurrently while contacting the bladder cell with the baculoviral vector. The transfection agent may be, for example, poly-L-lysine, Clorpaction WCS-90, dodecyl-B-dd-maltoside (DDM), or sodium dodecyl sulfate (SDS), or any combination thereof.

In keeping with the methods as described herein, the invention provides variouses uses of baculoviral vectors.

Other aspects and features of the present invention will become apparent to those of ordinary skill in the art upon review of the following description of specific embodiments of the invention in conjunction with the accompanying figures and tables.

### BRIEF DESCRIPTION OF THE DRAWINGS

The figures and tables, which illustrate, by way of example only, embodiments of the present invention, are as follows.

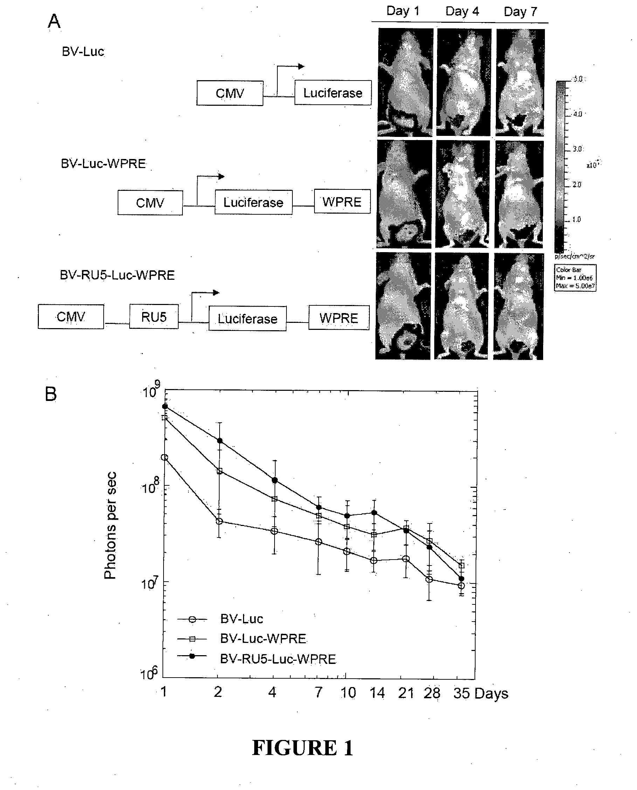

**Figure 1****. Baculoviral transduction of mouse bladder after intravesical instillation in balb/c nude mice.** (A). Bioluminescence images of luciferase reporter gene expression in representative animals transduced with 3 different baculoviral vectors. Heat map represents the transgene expression area and color represents the intensity. The schematic structures of baculoviral vector expression cassettes are shown on the left. Abbreviations: CMV: the human cytomegalovirus immediate-early gene promoter and enhancer; WPRE: the woodchuck hepatitis virus post-transcriptional regulatory element; RU5: R segment and part of the U5 sequence of long terminal repeat from the human T-cell leukemia virus type 1. (B). Time course analysis of luciferase reporter gene expression. *In vivo* gene expression levels are quantified by measuring bioluminescence signals. The data represent the mean + s.d., n = 3 per group.

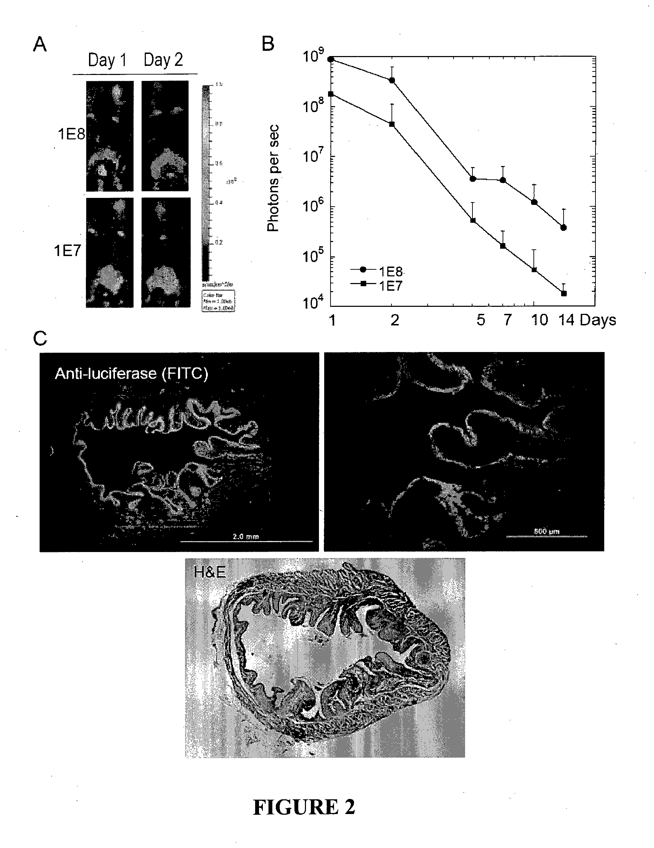

**Figure 2****. Baculoviral transduction of mouse bladder after intravesical instillation in C57L/B6 mice.** (A) Bioluminescence images of luciferase reporter gene expression in representative animals. Mice were transduced with the baculoviral vector BV-RU5-Luc-WPRE at a dose of 10⁸ or 10⁷ viral particles per mouse. (B) Time course analysis of luciferase reporter gene expression. *In vivo* gene expression levels are quantified by measuring bioluminescence signals. The data represent the mean + s.d., n = 3 per group. (C) Immunostaining with antibodies against luciferase protein to demonstrate baculoviral transduction in the bladder. The tissue sections were collected 24 hours after baculoviral transduction. A low-magnification image and a high-magnification image are shown. Hematoxylin and eosin staining is included to show the structure of normal bladder after baculoviral transduction.

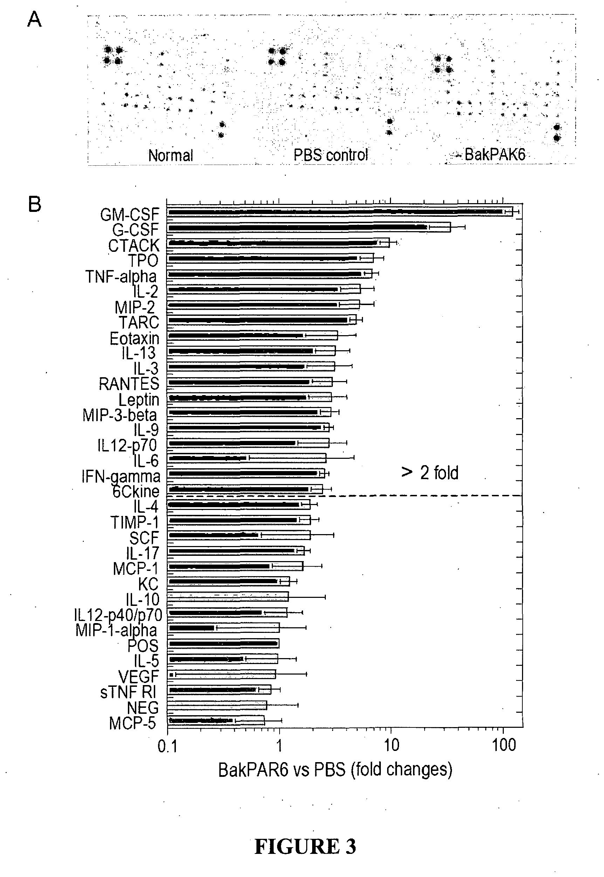

**Figure 3****. Baculoviral transduction up-regulates cytokine/chemokine expression in the bladder.** (A) Representative blot images of samples collected from C57L/B6 mice receiving no intravesical instillation (normal), intravesical instillation of PBS and BacPAK6 respectively. Bladders were harvested 48 hours after instillation and homogenized. The supernatants were used to probe the cytokine/chemokine antibody arrays. (B) Relative up-regulation of cytokines and chemokines in the mouse bladder upon baculoviral transduction. Densitometric data were analyzed with the RayBio® Analysis Tool. Fold changes (average signal intensity difference) are shown. The data represent the mean + s.d., n = 3 per group.

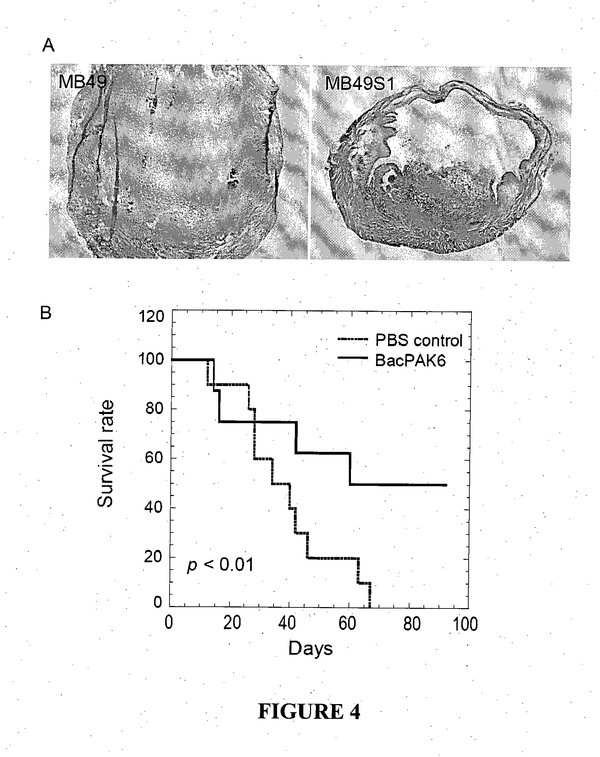

**Figure 4****. Anti-tumor effects of baculoviral transduction.** (A) Tumor development in the bladder after intravesical inoculation of mouse bladder cancer cells. Two types of mouse bladder cancer cells, MB49 and MB49S 1 (a subclone of MB49 cells), were tested at 10⁵ cancer cells per animal. The bladders were harvested at 14 day post-inoculation. H&E staining shows that the MB49S1 tumor has a slow growth rate compared to the tumor formed by inoculation of parental MB49 cells. (B) BacPAK6 transduction prolongs survival of bladder tumor-bearing mice. Intravesical instillation of BacPAK6, a baculoviral vector without mammalian gene expression cassette, was performed 7 days after inoculation of 10⁵ MB49S1 cells. Survival curves till day 95 are shown. n = 10 per group. The statistical analysis was performed using the log rank test.

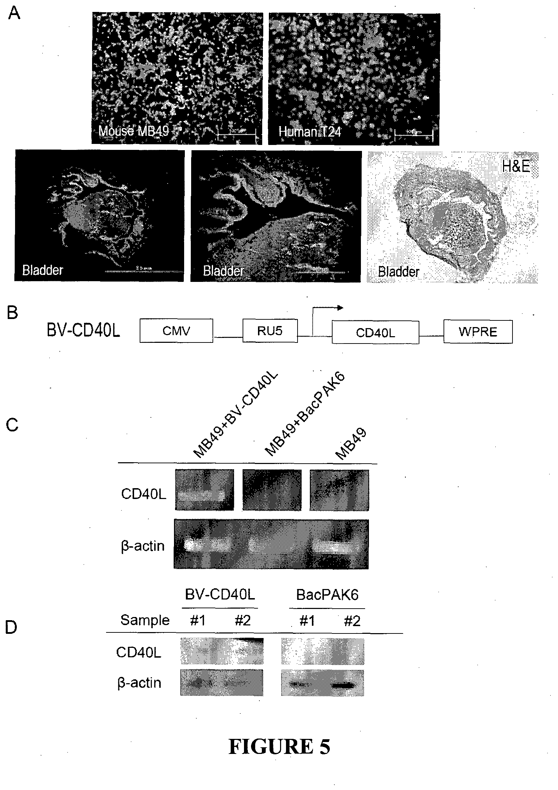

**Figure 5****.** Baculoviral vectors mediate CD40L expression in bladder cancer cells. Transduction efficacy of baculorviral vector in bladder cancer model. (A) Baculovirus is capable of transducing bladder cancer cells *in vitro* and *in vivo.* The baculoviral vector BV-RU5-Luc-WPRE was used to transduce mouse MB49 bladder cancer cells, human T24 bladder cancer cells, and mouse orthotopic bladder tumors formed by inoculating MB49 cells. Luciferase reporter gene expression was demonstrated by immunostaining with antibodies against the luciferanse protein in cells and tissue sections collected 24 hours after baculorvirus transduction. H&E staining shows bladder tumor growth. (B) Schematic structure of a baculoviral vector expression cassette for CD40L. (C) RT-PCR demonstrates CD40L expression in MB49 cells 24 hours after *in vitro* BV-CD40L transduction. (D) CD40L expression in the mouse MB49 bladder tumor as demonstrated by Western blotting. Intravesical instillation of BV-CD40L was performed 3 days after inoculation of 10⁵ MB49 cells. The bladders were collected 24 hours after BV-CD40L transduction.

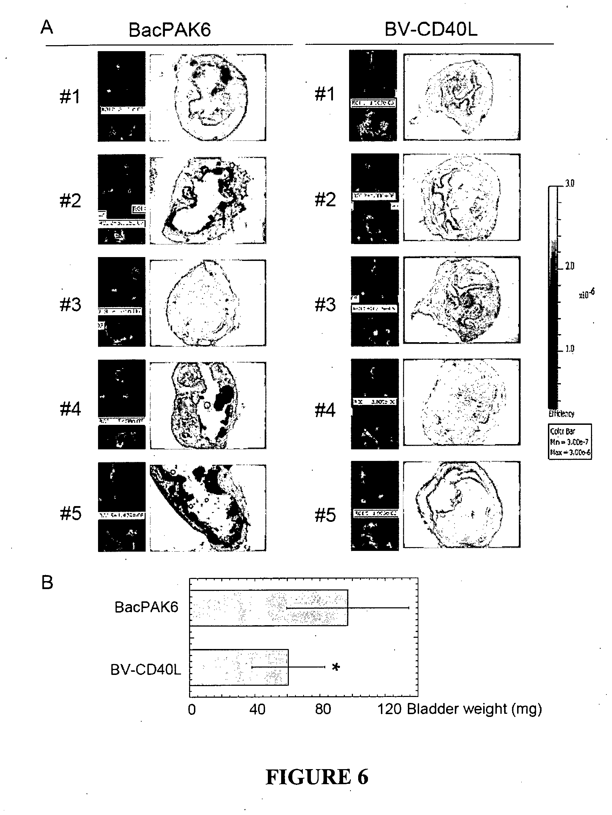

**Figure 6****. Anti-tumor effects of baculovirus-mediated CD40L expression.** Intravesical instillation of BV-CD40L was performed 3 days after inoculation of 2x 10⁴ DiR-labeled MB49 cells. Intravesical instillation of BackPAK6 was included as a transduction control. Bladders were collected 1 week after baculoviral transduction for analysis. (A) CD40L expression slows tumor growth. DiR fluorescence images show initial tumor burden in each animal and H&E staining shows tumor development. (B) Bladder weight difference. The data represent mean + s.d., n = 5 per group. * *p* < 0.05 versus the BacPAK6 group by Student t-test.

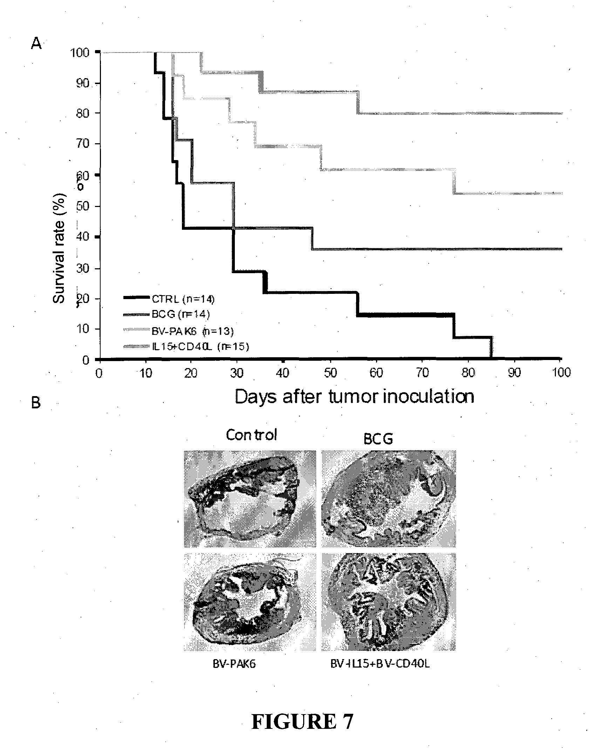

**Figure 7****. Effects of co-delivery of CD40L and IL-15 on the survival of bladder cancer-bearing mice. (A)** Co-delivery of CD40L and IL-15 by baculoviral vectors prolongs survival of bladder tumor-bearing mice. Intravesical instillation of the baculoviral vectors was performed 2 days after inoculation of 10⁵ MB49 cells. Survival curves till day 100 are shown. (B) H&E staining shows that the MB49 tumor was totally stopped in one mouse treated with the baculoviral vectors expressing CD40L and IL-15.

### DETAILED DESCRIPTION

The methods and uses described herein relate to the discovery that baculoviral vectors can be used in bladder cancer therapy, including to reduce or prevent recurrent cancer in a subject who has had bladder cancer. The baculoviral vector may, without any transgene insertion, act to stimulate an anti-tumor immune response. A therapeutic nucleic acid may be included in the baculoviral vector in order to increase or supplement an anti-tumor response. Under certain conditions, including under intervesical delivery, the baculoviral vector may preferentially transducer bladder tumor cells without transducing healthy non-cancer bladder cells.

As will be appreciated, transduction of a cell by a virus or virus vector refers to the delivery of the viral vector or viral genome into the cell, i.e. infection of the cell by the viral nucleic acid material. In the case of transduction of a mammalian cell by a baculoviral vector, transduction does not refer to product infection or the ability of the viral genome to be replicated within the cell.

The bladder cell that is to be contacted may be any bladder cell. The bladder cell may be an *in vitro* bladder cell, including a cell in tissue culture, such as a cell from an established bladder cell line, a primary bladder cell in tissue culture, an explanted bladder cell from a subject, a transformed or immortalised bladder cell. The bladder cell may be an *in vivo* bladder cell in a subject, including in a mammalian subject, including a human subject. For example, the bladder cell may be in a subject in need of treatment of bladder cancer. The bladder cell may be a transgenic bladder cell, including in an *in vitro* or *in vivo* context, including for example an animal model comprising a transgenic bladder cell.

The bladder cell may be a healthy, i.e. non-cancerous, bladder cell, or it may be a bladder cancer cell including a bladder cancer cell in a tumor or excised from a tumor, or it may be a bladder cell that is pre-disposed to become cancerous, including for example a bladder cell carrying a mutation that predisposes the bladder cell to become cancerous.

The baculoviral vector contacted with the bladder cell may be any baculoviral vector. Baculoviral vectors are known in the art, and the most commonly used baculoviral vectors are derived from *Autographa californica multiple nucleopolyhedrovirus* (AcMNPV). Baculovirus is an insect DNA virus has the ability to enter mammalian cells without replicating or causing toxicity to the transduced cell. Baculovirus has broad tropism in both proliferating and non-proliferating, quiescent cells and, with the support of a mammalian-active promoter, is capable of efficiently transferring genes of interest to diverse mammalian cell types *in vitro* and *in vivo.* The virus can enter mammalian cells but cannot express its own genes from insect-specific promoters; thus, baculoviruses are unable to replicate and express viral proteins in vertebrate cells, thus will not provoke immune responses as a consequence of *in vivo* expression of virally encoded genes, recombine with pre-existing viral materials nor assist the replication of other viruses in the human body. Infection of baculoviruses in mammalian cells causes no visible cytopathic effects, even at a high MOI (Shoji et al., 1997).

Another attractive advantage of using baculovirus AcMNPV, for example as a gene delivery vector, is the large cloning capacity conferred by its 130 kb viral genome, which may be favorably used to deliver a large functional gene or multiple genes from a single vector. In human cells, baculoviral vectors carrying a mammalian expression cassette may be effective in mediating transient expression, as the transgenic vectors typically do not integrate into the genome of the transduced cells. Thus, baculoviral vectors are ideally suited for applications requiring short-term, high level transgene expression and pose low risk of insertional mutagenesis.

Another important advantage of baculoviral transduction is the absence of pre-existing immunity against the insect virus in humans (Wang S et al., 2010), since this virus is not infectious to humans. Strauss *et al.* have reported that the prevalence of neutralizing antibodies against adenovirus type 5 was 65% in human serum samples, whereas none of the serum samples was positive for baculovirus neutralizing antibodies (Strauss R, et al, 2007). In the same study, pre-existing adenovirus-specific T cells were detectable but there were no pre-existing baculovirus-specific T cells in humans. Compared to adenovirus, baculovirus has relatively low immunogenicity as indicated by the induction of lesser number of virus-specific T-cells (Strauss R, et al, 2007).

As well, unlike many other gene therapy viral vectors, baculoviral vectors can be produced in serum-free cell culture medium, which eliminates the potential hazard of serum contamination with viral and prion agents from the serum-donating animal.

The baculoviral vector itself, without any transgene, may be the nucleic acid to be delivered to the bladder cell. That is, delivery may result in the cell being transduced by, or taking up, the baculoviral vector nucleic acid material.

In other embodiments, the baculoviral vector may include a transgene coding sequence encoding an expression product that is to be expressed in the bladder cell. It will be appreciated that the transgene sequence encoding an expression product will be operably linked to all the necessary regulatory sequences, including a promoter region, to effect the desired expression profile of the expression product within the bladder cell. A first nucleic acid sequence is operably linked with a second nucleic acid sequence when the sequences are placed in a functional relationship. For example, a coding sequence is operably linked to a promoter if the promoter activates and drives the transcription of the coding sequence. Thus, baculoviral vector that is contacted with the bladder cell may further comprise a transgene for expression in the bladder cell operably linked to a promoter that drives expression of the transgene in the bladder cell.

In some embodiments, the promoter that is operably linked to the transgene coding sequence comprises the human cytomegalovirus immediate early promoter. In some embodiments, the promoter comprises the human cytomegalovirus immediate early promoter having the sequence set forth in SEQ ID NO: 1.

In some embodiments, the baculoviral vector comprises a promoter that essentially consists of the human cytomegalovirus immediate early promoter having the sequence set forth in SEQ ID NO: 1. In some embodiments, the promoter consists of the human cytomegalovirus immediate early promoter having the sequence set forth in SEQ ID NO: 1.

As used herein, "consists essentially of' or "consisting essentially of' means that the nucleic acid sequence includes one or more nucleotide bases, including within the sequence or at one or both ends of the sequence, but that the additional nucleotide bases do not materially affect the function of the nucleic acid sequence, for example to function as a promoter to drive expression of an operably linked coding sequence in a bladder cell.

In other embodiments, the promoter is a human cytomegalovirus immediate early promoter having at least 80%, at least 85%, at least 90%, at least 95%, or at least 99%, sequence identity to SEQ ID NO: 1, while still retaining the ability to direct gene expression in the bladder cell.

The baculoviral vector may further comprise post-transcriptional regulatory elements, including for example post-transcriptional regulatory elements from the woodchuck hepatitis virus, in the 3' untranslated region of the transgene. In some embodiments, the baculoviral vector comprises post-transcriptional regulatory elements from the woodchuck hepatitis virus comprising the sequence as set forth in SEQ ID NO: 2.

In some embodiments, the baculoviral vector comprises a post-transcriptional regulatory element from the woodchuck hepatitis virus that consists essentially of the sequence as set forth in SEQ ID NO: 2. In some embodiments, the baculoviral vector comprises post-transcriptional regulatory element from the woodchuck hepatitis virus that consists of the sequence as set forth in SEQ ID NO: 2.

In other embodiments, the In some embodiments, the baculoviral vector comprises a post-transcriptional regulatory element from the woodchuck hepatitis virus having at least 80%, at least 85%, at least 90%, at least 95%, or at least 99%, sequence identity to SEQ ID NO: 2, while still retaining the ability to direct gene expression in the bladder cell.

The baculoviral vector may further comprise the R segment and at least a portion of the U5 sequence of the long terminal repeat from the human T-cell leukemia virus type 1, in the 5' untranslated region of the transgene, including for example wherein the R segment and at least a portion of the U5 sequence of the long terminal repeat from the human T-cell leukemia virus type 1 comprising the sequence set forth in SEQ ID NO: 3.

In some embodiments, the baculoviral vector comprises the upstream 5'regulatory region of R segment and at least a portion of the U5 sequence of the long terminal repeat from the human T-cell leukemia virus type 1 that consists essentially of the sequence as set forth in SEQ ID NO: 3. In some embodiments, the baculoviral vector comprises comprises the upstream 5'regulatory region of R segment and at least a portion of the U5 sequence of the long terminal repeat from the human T-cell leukemia virus type 1 that consists of the sequence as set forth in SEQ ID NO: 3.

In other embodiments, the In some embodiments, the baculoviral vector comprises post-transcriptional regulatory elements from the woodchuck hepatitis virus having at least 80%, at least 85%, at least 90%, at least 95%, or at least 99%, sequence identity to SEQ ID NO: 3, while still retaining the ability to direct gene expression in the bladder cell.

The baculoviral vector may be so modified using standard techniques that will be known to a skilled person, such as PCR and molecular cloning techniques. For example, baculovirus can be readily modified using commercially available cloning and expression systems such as the BAC-TO-BAC™ Baculovirus Expression system (Gibco BRL, Life Technologies, USA).

In addition to the regulatory regions, the baculovirus may include a transgene coding sequence. The term transgene refers to a gene which is foreign to baculovirus, such that, for example, reference to expression of a transgene by a baculoviral vector refers to expression of a gene that is foreign to the baculoviral genome. The transgene coding sequence may encode any expression product desired to be expressed in the bladder cell. For example, the transgene may encode a therapeutic protein or RNA, a selectable protein or RNA marker, a detectable reporter protein or RNA or a protein that provides resistance to selective environmental conditions for the bladder cell. The transgene may also encode a regulatory RNA, for example an miRNA to regulate expression of a gene of the bladder cell.

For example, the transgene may encode a therapeutic expression product for treating bladder cancer. The transgene may thus be a therapeutic transgene, including any gene having clinical usefulness, such as a gene encoding a gene product such as an RNA or protein that is involved in cancer prevention or treatment, or a gene having a cell regulatory effect that is involved in cancer prevention or treatment. The gene product may substitute a defective or missing gene product, protein, or cell regulatory effect in the subject, thereby enabling prevention or treatment of bladder cancer.

Thus, a therapeutic expression product includes a protein or peptide that when expressed in the bladder cell, has a therapeutic effect on the cell, or which effects a desired result within the bladder cell.

The therapeutic expression product may be an RNA molecule, for example an antisense RNA, an miRNA or a small interfering RNA (siRNA) molecule, that results in regulation of gene expression in the bladder cell that provides clinically useful regulation. The antisense RNA, miRNA or siRNA may be involved in down-regulating or inhibiting or reducing expression of a gene involved in tumorogenesis or tumor growth.

For example and without limitation, the therapeutic transgene may encode a protein that is CD40L or IL-15. In one example, therapeutic transgenes encoding CD40L and IL-15 may be used together in the same subject for therapeutic effect. The CD40L and IL-15 transgenes may be included together in the same baculoviral vector, or may be included in different baculoviral vector that are then delivered to the same subject.

In order to deliver the nucleic acid molecule to the bladder cell, the bladder cell is contacted with the baculoviral vector. Contacting may comprise, for example, addition of the baculoviral vector to tissue culture medium containing the bladder cell so that the bladder cell is transduced by the baculoviral vector. Contacting may comprise administering the baculoviral vector to a subject.

Thus, the bladder cell to which the nucleic acid molecule is to be delivered may be *in vivo* in a subject in need of treatment or prevention of bladder cancer, including a mammal, including a human.

The method may therefore be performed in the context of an *in vivo* bladder cell that is cancerous, that is part of a tumor, that is predisposed to become cancerous, or which is to be prevented from becoming cancerous.

Thus, contacting the bladder cell with the baculoviral vector includes administering an effective amount of the baculoviral vector to the subject. The term "effective amount" as used herein means an amount effective, at dosages and for periods of time necessary to achieve the desired result, for example, to treat or prevent bladder cancer, including preventing or reducing the risk of recurrence of bladder cancer in a subject that has previously had bladder cancer, including superficial bladder cancer, including a TCC.

As used herein, "treat" or "treatment" in respect of a bladder cancer refers to an approach for obtaining beneficial or desired results, including clinical results. Beneficial or desired clinical results can include, but are not limited to, alleviation or amelioration of one or more symptoms or conditions, diminishment of extent of disease, stabilization of the state of disease, prevention of development of disease, prevention of spread of disease, prevention of recurrence of disease, delay or slowing of disease progression or recurrence, delay or slowing of disease onset, amelioration or palliation of the disease state, and remission (whether partial or total). "Treating" can also mean prolonging survival of a subject beyond that expected in the absence of treatment. "Treating" can also mean inhibiting the progression or recurrence of disease, slowing the progression or recurrence of disease temporarily, although more preferably, it involves halting the progression or recurrence of the disease permanently.

Recombinant vectors derived from the insect baculovirus *Autographa californica multiple nucleopolyhedrovirus* (AcMNPV) have been suggested as vectors useful for gene therapy, including for cancer gene therapy (Hofmann C et al., 1995; Kost TA et al., 2005; Hu YC, 2008, 2010; Wang S et al., 2010). Takaku et al. have the activation of NK cell-dependent antitumor immunity by baculovirus (Kitajima M et al., 2008a). Baculovirus-induced antitumor action may possibly involve acquired immunity by enhancing tumor-specific cytotoxic T lymphocyte (CTL) responses and tumor-specific antibody production (Kitajima M et al., 2008b; Suzuki T et al., 2010). Thus, the immunostimulatory properties of baculovirus can be employed in the described methods for cancer immunotherapy.

The baculoviral vector may be administered to the subject using standard techniques known in the art. The baculoviral vector may be administered systemically, or may be administered by intravesical instillation into the bladder.

As will be appreciated, the bladder is a hollow organ, allowing nonsurgical intravesical drug administration through a urethral catheter and evaluation of treatment efficacy by the mean of endoscopy. Taking advantage that intravesically delivered therapeutics act locally with limited systemic exposure and that superficial bladder cancer is easily accessible.

With *in vivo* baculoviral transduction of mammalian cells, inactivation of systemically delivered baculoviral vectors may result as a consequence of virus recognition by serum complement proteins, a major component of innate immune system (Hofmann C et al., 1998). In immunocompetent animals, systemically delivered baculoviral vectors may fail to be expressed. High titers of baculovirus have been used to overcome the ability of the complement to neutralize the virus; however, this approach may still not result in baculovirus-mediated transgene expression (Kitajima M et al., 2008a). Thus, bladder cancer therapy can be preformed with intravesical catheterization through the urethra to avoid many drawbacks to systemic virus administration such as virus inactivation by serum complement.

As demonstrated in the presently described methods, intravesically administrated baculoviruses can display a strong immunostimulatory capacity to induce the expression or promote the release of inflammatory cytokines. The finding is consistent with previous studies that found that after injected into the animal body, baculovirus can elicit protective innate immune responses. Baculovirus can induce adaptive immunity as well, featured as an immune response specifically directed against the products of viral genes (Pieroni et al. 2001; Abe et al. 2005).

Without being limited by theory, the above immunostimulatory effects can possibly be harnessed therapeutically to inhibit tumor growth. This hypothesis is supported by the observation that intravesically delivered baculoviruses that do not express any transgenes, but are able to prolong survival of immunocompetent mice bearing established orthotopic bladder cancer. This syngeneic model system provides intact immune functions, allowing for the studies of therapeutic baculoviral based treatments against bladder cancer.

In order to increase efficiency of uptake by bladder cells upon intravesical instillation of the baculoviral vector, the method may further comprise use of a transfection agent. For example, the bladder may first be treated with a transfection agent for a given period of time, prior to contacting the baculoviral vector with the bladder cell via intravesical instillation.

Transfection agents are known in the art; for example, the transfection agent may be poly-L-lysine, Clorpaction WCS-90, dodecyl-B-dd-maltoside (DDM), or sodium dodecyl sulfate (SDS), or any combination thereof.

However, it was observed that performing the method without any pretreatment of the bladder cells with a transfection agent may alter the uptake of the baculoviral vector by healthy (non-cancerous) bladder cells in comparison to bladder cancer cells. Thus, in some embodiments, the method omits any use of transfection agent, in order to preferentially target bladder cancer cells over healthy bladder cells.

The concentration and amount of baculoviral vector to be administered will vary, depending on the bladder cancer to be treated, the type of transgene included in the baculoviral vector, the mode of administration, other concurrent treatments, and the age and health of the subject. Survival and cancer prevention or treatment may be improved by varying the frequency of intravesical instillation.

As indicated above, the efficacy of the baculoviral vector may be improved by including a therapeutic transgene in the baculoviral vector.

To aid in administration, the baculoviral vector may be formulated as an ingredient in a pharmaceutical composition.

Therefore, there is also provided a pharmaceutical composition comprising baculoviral vector as described above, and optionally a pharmaceutically acceptable diluent. Such pharmaceutical compositions may be for use in treating bladder cancer, as described above.

The compositions may routinely contain pharmaceutically acceptable concentrations of salt, buffering agents, preservatives and various compatible carriers. For all forms of delivery, the baculoviral vector formulated in a physiological salt solution.

The proportion and identity of the pharmaceutically acceptable diluent may be determined by chosen route of administration, compatibility with live cells and live virus particles, and standard pharmaceutical practice. Generally, the pharmaceutical composition will be formulated with components that will not kill or significantly impair the biological properties of the baculoviral vector.

The pharmaceutical composition can be prepared by known methods for the preparation of pharmaceutically acceptable compositions suitable for administration to subjects, such that an effective quantity of the baculoviral vector, and any additional active substance or substances, is combined in a mixture with a pharmaceutically acceptable vehicle. Suitable vehicles are described, for example, in Remington's Pharmaceutical Sciences (Remington's Pharmaceutical Sciences, Mack Publishing Company, Easton, Pa., USA 1985). On this basis, the pharmaceutical compositions include, albeit not exclusively, solutions of the baculoviral vector, in association with one or more pharmaceutically acceptable vehicles or diluents, and contained in buffer solutions with a suitable pH and iso-osmotic with physiological fluids.

The dose of the pharmaceutical composition that is to be used depends on the particular condition being treated, the severity of the condition, the individual subject parameters including age, physical condition, size and weight, the duration of the treatment, the nature of concurrent therapy (if any), the specific route of administration and other similar factors that are within the knowledge and expertise of the health practitioner. These factors are known to those of skill in the art and can be addressed with minimal routine experimentation.

Also contemplated are various uses of the described baculoviral vector. Thus, there is provided a baculoviral vector for use in intravesical instillation in a subject in need of treatment of bladder cancer, use of a baculoviral vector for treating bladder cancer in a subject in need of such treatment by intravesical instillation of the baculoviral vector, use of a baculoviral vector for manufacture of a medicament for treating bladder cancer in a subject in need of such treatment by intravesical instillation of the baculoviral vector, use of a baculoviral vector for intravesical instillation in a subject in need of treatment of bladder cancer, and use of a baculoviral vector for manufacture of a medicament for intravesical instillation in a subject in need of treatment of bladder cancer.

The present methods and uses are further exemplified by way of the following non-limiting examples.

### EXAMPLES

### EXAMPLE 1

Use of insect baculovirus-based vectors for bladder cancer therapy was investigated. It was first demonstrated that intravesically delivered baculoviral vectors could transduce the normal mouse bladder effectively. A new recombinant baculoviral vector constructed by incorporating into the viral genome a mammalian expression cassette containing the human cytomegalovirus immediate-early gene promoter, the woodchuck hepatitis virus post-transcriptional regulatory elements, and the R segment and part of the U5 sequence of long terminal repeat from the human T-cell leukemia virus type 1 provided the highest *in vivo* transduction efficiency among 3 different baculoviral vectors tested. It was then investigated whether the viral transduction alone could stimulate antitumor immunity. Using murine cytokine/chemokine antibody arrays to analyze bladder tissue extracts collected from mice receiving intravesical administration of baculoviruses without any transgenes, 59% of the proteins in the array (19 out of 32) showed a >2-fold increase in expression, with granulocyte-macrophage colony-stimulating factor, granulocyte colony-stimulating factor, and cutaneous T cell-attracting chemokine as the top three up-regulated proteins. More importantly, the treatment significantly prolonged survival of orthotopic bladder cancer-bearing mice, with 50% of the animals surviving beyond six months. When baculoviral vectors were used to deliver the CD40 ligand gene intravesically into the bladder of mice with aggressive orthotopic bladder tumor progression, preferential transduction of the tumor followed by reduction of tumor growth and average bladder weight was detected.

### Materials and Methods

**Baculovirus preparation:** Recombinant baculoviral vectors, CMV-Luc, CMV-Luc-WPRE and CMV-RU5-Luc-WPRE, were constructed using BAC-to-BAC™ baculovirus expression system according to the manufacturer's manual (Invitrogen). CMV-Luc contains a luciferase gene under the control of human cytomegalovirus (CMV) early promoter. CMV-Luc-WPRE has an extra woodchuck hepatitis virus posttranscriptional regulatory element (WPRE) at the 3' untranslated region (UTR). CMV-RU5-Luc-WPRE has another regulatory element RU5, the R segment and part of the U5 sequence of long terminal repeat from the human T-cell leukemia virus type 1, at the 5' UTR. These luciferase-expressing viruses were produced by transfection of Sf9 insect cells using corresponding bacmids. BV-CD40L (CD40 ligand) virus, which has the mouse CD40L gene (Invivogen) under the control of CMV promoter with RU5 at 5' UTR and WPRE at 3' UTR, was produced by homologous recombination after co-transfection of Sf9 insect cells with pBacPAK9 transfer vector containing the expression cassette and linearized AcMNPV viral DNA (Clonetech). BV-IL-15 (interleukin 15) virus was constructed in a similar manner to BV-CD40L, but using the mouse IL15 gene. BacPAK6, the parental virus with the lacZ gene driven by viral polyhedrin promoter, was obtained from Clonetech. Recombinant baculoviruses were amplified in Sf9 cells at an MOI of 0.1 and the virus-containing supernatant was collected 3 days after virus infection. Viruses were pelleted down at 28,000g for 1 hour and re-suspended in PBS.

**Cell lines and *in vitro* baculoviral transduction:** Sf9 insect cells (Invitrogen) were maintained in sf-900 III serum free medium (Invitrogen). Murine bladder carcinoma cell line MB49 was a gift from Dr. Esuvaranathan (National University Hospital, Singapore) and the human bladder carcinoma cell line T24 was purchased from ATCC. The cell lines were maintained in RPMI 1640 supplemented with 10% fetal bovine serum (Hyclone, Logan, UT), 2 mM L-Glutamine, 1% penicillin and streptomycin (Sigma) at 37°C and 5% CO2. To obtain clonal MB49 variants with altered growth behavior to further our understanding of the molecular mechanisms underlying bladder cancer growth, wild-type MB49 cells were transfected with pRC2/CMV-Luc, a plasmid with a luciferase gene driven by the CMV promoter and a neomycin resistance gene under the control of the SV40 promoter. The transfected cells were selected with G418 at a concentration of 700 µg/ml for 2 weeks. A subclone, MB49S1, with a high tumor establishment rate and a relatively slow growth rate in the mouse bladder was also used in the current study. For *in vitro* baculoviral transduction experiments, the tumor cells were incubated with baculoviral vectors at an MOI of 100 overnight at 37 °C. Transgene expression level was determined 24 hours after transduction.

**Animals, animal model and in vivo baculoviral transduction:** Adult female C57BL/6 mice and adult female balb/c nude mice (weight 20 g, aged 5-6 weeks) were used. Orthotopic bladder tumors were generated on the luminal surface of the bladder by intravesical instillation of syngeneic MB49 cells in C57BL/6 mice. Before tumor inoculation, MB49 cells were pre-labeled with a lipophilic, near-infrared fluorescent day DiR (20 ng/ml overnight, Caliper Life Sciences) to facilitate *in vivo* monitoring of tumor take rate. A 24-guage catheter (BD Medical) was introduced into the bladder of anesthetized female mice through urethra for intravesical instillation. After the residual urine was squeezed out, the bladder was infused with 100 µl of 10 µg/ml poly-L-lysine (PLL, mol. Wt. 70,000-150,000, Sigma). The PLL solution was retained in the bladder for 30 min before being squeezed out. The bladder was then washed with 100 µl of PBS. After the pretreatment, 100 µl of MB49S1 or MB49 bladder cancer cells in PBS were instilled and retained in the bladder for 1 hour. A dose of 1x 10⁵ cancer cells per animal was used in most experiments, except in the CD40L and/or IL-15 therapy studies, where a low dose of 2x 10⁴ cells per animal was used. Thereafter, the catheter was removed and the bladder was evacuated by spontaneous voiding. Tumor take rate was examined 24 hours after intravesical instillation using the IVIS 100 *in vivo* imaging system coupled with a cool CCD camera and the ICG filter (Caliper Life Sciences). Images and the fluorescent signals were acquired and analyzed with the Xenogen living imaging software v2.5. Animals successfully implanted with MB49 cells and with similar tumor burden were selected and used in the following experiments. For *in vivo* baculoviral transduction in the bladder, mice were anaesthetized and catheterized. Baculoviral vectors in PBS (1 x 10⁸ pfu viral particles in 100 µl) was instilled after a 30-minute poly-L-lysine treatment and retained for 1 hour.

To evaluate *in vivo* transduction efficiency in the bladder, mice with or without orthopic tumor implantation were transduced with a baculoviral vector with the luciferase reporter gene and imaged in the supine position with the IVIS 100 imaging system coupled with a cool CCD camera and an emission filter of 560 nm (Caliper Life Sciences) 15 minutes after i.p. injection of 150 mg/kg luciferin (Promega). Images and the luminescent signals were acquired and analyzed with the Xenogen living imaging software v2.5 and quantified as photons per second.

To evaluate therapeutic efficacy, *in vivo* baculorviral transduction of the bladders was performed 7 days after orthopic tumor implantation. The mice were randomized to control or treatment groups. After the mice were re-anaesthetized and re-catheterized, BacPAK6 or BV-CD40L or BV-IL-15 viruses were instilled. Animals were either euthanized 7 days later for histological analysis or observed for 6 months for signs and symptoms of bladder cancer (hematuria and weight loss) and viability status.

All handling and care of animals were carried out according to the Guidelines on the Care and Use of Animals for Scientific Purposes issued by the National Advisory Committee for Laboratory Animal Research, Singapore.

**Histological analysis and immunostaining:** For histological examination, bladders were harvested and fixed in 4% PFA overnight, suspended in 30% sucrose, and embedded in a tissue freezing medium. Cryostat sections at 10 µm were prepared and stained with hematoxylin & eosin (H & E). For immunostaining, the tissue sections were washed twice with Tris-buffered saline Tween-20 (TBST) and incubated in 0.025% Triton X-100 for 10 min. The tissue sections were then incubated in 5% BSA for 1 h to block nonspecific binding. The rabbit polyclonal anti-luciferase antibody (Abcam, 1:100) was applied overnight at 4°C. After 3 times washing with TBST, slides were incubated with the secondary antibody, FITC conjugated goat anti-rabbit IgG (1:200), for 1 hour at room temperature.

**Cytokine/chemokine expression:** Mice were euthanized 48 hours after intravesical instillation of PBS or BacPAK6. Bladders were harvested and weighed. A tissue lysis buffer (Fermentas, Maryland, USA) with a protease inhibitor cocktail (Calbiochem,Merck, Darmstadt, Germany) was added at 50 mg tissue per ml and bladders were homogenized by sonication. Bladder homogenates were then centrifuged at 16,000 x g for 30 min at 4°C and the supernatants collected. Protein concentrations of the supernatants were determined by the Biorad protein assay method (Biorad, California, USA). An aliquot of the supernatant containing 80 □µg of total protein concentration was loaded onto the Mouse Cytokine Array 2.1 (Raybiotech, Norcross, GA) to measure expression levels of cytokines and chemokines according to the manufacturer's instructions. RayBio™ Analysis Tool (Raybiotech) was used to correlate the average signal intensities to relative expression levels of cytokines.

### Results

**Baculoviral vectors effectively transduce the mouse bladder after intravesical instillation:** It was first assessed whether baculovirus is able to transduce the bladder in immunodeficient nude mice. Three different recombinant baculorviral vectors containing a firefly luciferase gene were tested after intravesical instillation into the bladder and *in vivo* transduction efficiency was monitored using the IVIS living animal imaging system (Figure 1A). All three vectors were effective in transducing the mouse bladder after pre-treating the organ with poly-L-lysine. One of the baculoviral vectors, BV-RU5-Luc-WPRE, that contains two viral transcriptional regulatory elements WPRE and RU5, provided the highest transgene expression level in the bladder. While decreasing over time, the expression levels provided by the three vectors remained significantly higher than a background level for at least 35 days. (Figure 1B)

In immuno-competent C57BL/6 mice (Figures 2A, 2B), although the initial expression level provided by BV-RU5-Luc-WPRE was similar to that observed in immunodeficient nude mice, the level dropped quickly and the detectable transgene expression lasted for approximately 2 weeks only. The difference in transgene expression between the two types of mice indicates a strong immune response to baculoviral transduction. Baculovirus-mediated transgene expression in the bladder was dosage-dependent, as evidence by the observation that the luciferase expression level at day 1 in C57BL/6 mice treated with 10⁷ viral particles per mouse was approximately ¼ of that treated with 10⁸ viral particles per mouse (Figure 2A, B). Immunohistological staining with an antibody against the luciferase protein confirmed that baculovirus-mediated transgene expression was confined to the superficial bladder epithelium (Figure 2C).

**Baculoviral transduction alone is capable of retarding bladder tumor growth:** Expression of cytokines and chemokines in the organ upon baculoviral transduction in immuno-competent C57BL/6 mice was investigated. BacPAK6, a baculoviral vector without mammalian gene expression cassette, was used for this purpose so as to avoid possible interference by transgene expression. Using an antibody array method, the up-regulation (defined as >2-fold increase in expression) of 59% the cytokines and chemokines was detected in a murine array (19 out of 32) in the bladder that received intravesical instillation of BacPAK6 two days ago as compared to the expression levels in the bladder that received PBS instillation (Figures 3A, B). The top 5 up-regulated proteins were granulocyte-macrophage colony-stimulating factor (GM-CSF, 123-fold increase), granulocyte colony-stimulating factor (G-CSF, 35-fold increase), cutaneous T cell-attracting chemokine (CTACK, 10-fold increase), thrombopoietin (TPO, 7-fold increase), and tumor necrosis factor alpha (TNF-alpha, 7-fold increase). A comparison between the normal mouse bladder without any treatment and the bladder receiving PBS instillation showed no significant difference in the expression levels of the cytokines and chemokines.

It was then investigated whether changes in expression levels of the cytokines and chemokines upon baculoviral transduction would affect bladder tumor growth. In the process to establish a syngeneic orthotopic mouse bladder cancer model to study the effects, it was noticed that MB49 cells grew quickly to occupy the whole lumen of the urinary bladder in 2 weeks even at a low inoculation dose (1x 10⁵ cancer cells per animal) (Figure 4A). The mice also died quickly due to aggressive tumor growth, with the first animal death between day 10 to 14 and the death of all animals in approximately 4 weeks (Günther JH, et al., 1999). These features of MB49 cells have made both performing accurate intravesical instillation and testing experimental therapeutics, especially immune therapeutics, targeted to treat established bladder tumors difficult. MB49S1 cells, a subclone of MB49 mouse bladder cancer line that showed a slower growth rate yet retained a high tumor establishment rate (Figure 4A), were therefore used to establish a bladder cancer model by intravesical instillation of the cells into the poly-L-lysine pretreated bladder in C57BL/6 mice.

One week after inoculation of 1x 10⁵ MB49S1 cells, the C57BL/6 mice with established tumors were randomly distributed into two groups (n = 10 per group). One group received single instillation of 10⁸ BacPak6 viral particles and another group received PBS as an instillation control. Significantly prolonged survival of the bladder tumor-bearing mice was observed after baculoviral stimulation in the bladder. While all animals in the control group died within 70 days, 50% of the animals in the treatment group were alive 6 months after tumor inoculation (Figure 4B, p < 0.01 in log-rank test). This finding demonstrates that up-regulation of cytokines and chemokines upon baculoviral transduction in the bladder is functional in mediating antitumor effects and may act to cure established bladder tumors.

**Baculoviral vectors can mediate high-efficiency gene transfer to bladder cancer cells and CD40L gene therapy for bladder cancer:** To assess whether baculoviral vectors can be used for delivering a transgene into bladder cancer, cultured mouse MB49 bladder cancer cells and human T24 bladder cancer cells were transduced *in vitro* with BV-RU5-Luc-WPRE. Immunostaining with an antibody against the luciferase protein confirmed high levels of luciferase expression in these tumor cells (Figure 5A). In C57BL/6 mice with orthotopic MB49 tumors, intravesical instillation of BV-RU5-Luc-WPRE resulted in obvious transgene expression in the tumors, as well as in the normal bladder epithelium (Figure 5). In the tumors, positive staining was observed not only in the surface layer of the tumors but also in inner tumor areas, indicating the penetration of the virus in tumors.

The baculoviral vector BV-CD40L was constructed by replacing the luciferase gene in the BV-RU5-Luc-WPRE expression cassette with the murine CD40 ligand (CD40L) gene, a gene encoding a type II transmembrane protein that functions as a potent T helper 1 immune stimulator. CD40L expression upon baculoviral transduction in MB49 cells *in vitro* and orthotopic MB49 tumors *in vivo* was confirmed using RT-PCR (Figure 5C) and Western blotting (Figure 5D), respectively.

To test the therapeutic effects of BV-CD40L, a bladder tumor model was established by inoculation of 2x 10⁴ DiR-labeled MB49 cells into the bladder of C57BL/6 mice. Successful tumor establishment was confirmed by monitoring DiR infrared fluorescence signals using the IVIS living animal imaging system (Figure 6A). At day 4 post-tumor inoculation, mice were randomly distributed into two groups (n = 5 per group) and treated via intravesical instillation of 10⁸ BV-CD40L viral particles per animal or 10⁸ BacPAK6 viral particles as a control. The 2^{nd} and 3^{rd} treatments in the two groups were performed on days 6 and 8 post-tumor inoculation. The mice were sacrificed on day 14 and the tumor development stage in the bladder was assessed by examining H&E stained bladder tissue sections (Figure 6A). In the control group, tumors had progressed to a late stage, with serious tumor necrosis and tumor invasiveness into the muscle layer in 4 out of 5 animals (#1, 2, 4, 5). Bladder urothelium was totally damaged and disorganized in these mice. In the treatment group, tumor mass was relatively smaller and there was no significant damage in the urothelium. Some tumors (#1 and 3) are still superficial and others (#2, 4 and 5) had grown from the layers of cells lining the lumen of the mouse bladder into the connective tissue below, but not into the muscle layer yet. Average bladder weight of this group was statistically significantly lower than that in the control group (*p* < 0.05, Figure 6B). These findings indicate that baculoviruses can possibly be used as an *in vivo* gene therapy vector to deliver therapeutic genes to treat aggressive growth bladder cancer.

**Effects of co-delivery of CD40L and IL-15 on the survival of bladder cancer-bearing mice:** In addition to the BV-CMV-RU5-Luc-WPRE and BV-CD40L viruses, BV-IL-15 was constructed by replacing the luciferase gene in the BV-RU5-Luc-WPRE expression cassette with the murine interleukin-15 (IL15) gene, a gene encoding a cytokine that induces proliferation of natural killer cells, cells that form part of the innate immune system. Therapeutic effects of BV-CD40L virus and BV-IL-15 virus were tested separately to confirm therapeutic gene expression and then co-delivered into the bladder of bladder cancer-bearing mice. Intravesical instillation of the baculoviral vectors was performed 2 days after inoculation of 10⁵ MB49 cells.

The results are seen in Figure 7. It was observed that co-delivery of CD40L and IL-15 by baculoviral vectors was able to prolong the survival of bladder tumor-bearing mice. (Figure 7A). In one mouse treated with the combination of baculoviral vectors expressing CD40L and IL-15, the MB49 tumor was totally stopped. (Figure 7B).

### Discussion

The immunostimulatory effects of baculovirus in mammals can possibly be harnessed therapeutically to inhibit bladder tumor growth. This hypothesis is supported by the observation that intravesically delivered baculoviruses that do not express any transgenes were able to prolong survival of immunocompetent mice bearing established orthotopic bladder cancer. This syngeneic model system provides intact immune functions, allowing the studies of therapeutic vaccines against bladder cancer. Although only half of the tumor-bearing animals were cured after just one injection of the non-transgenic baculoviral vector, the survival can be improved if the frequency of intravesical instillation is increased. The efficacy can also be improved by including a therapeutic gene into the baculoviral vector. In this regard, the anti-tumor effects of baculoviral vector-mediated CD40L expression were demonstrated in an animal model with aggressive growth bladder cancer.

Thus, these above-described results demonstrated, using a syngeneic orthotopic animal model of bladder cancer, that insect baculovirus can be used as a new agent for bladder cancer therapy with a dual function of therapeutic vaccination and therapeutic gene delivery.

All publications and patent applications cited in this specification are herein incorporated by reference as if each individual publication or patent application were specifically and individually indicated to be incorporated by reference. The citation of any publication is for its disclosure prior to the filing date and should not be construed as an admission that the present invention is not entitled to antedate such publication by virtue of prior invention.

As used in this specification and the appended claims, the singular forms "a", "an" and "the" include plural reference unless the context clearly dictates otherwise. As used in this specification and the appended claims, the terms "comprise", "comprising", "comprises" and other forms of these terms are intended in the non-limiting inclusive sense, that is, to include particular recited elements or components without excluding any other element or component. As used in this specification and the appended claims, all ranges or lists as given are intended to convey any intermediate value or range or any sublist contained therein. Unless defined otherwise all technical and scientific terms used herein have the same meaning as commonly understood to one of ordinary skill in the art to which this invention belongs.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

### References

1. Abe T, Takahashi H, Hamazaki H, et al. Baculovirus induces an innate immune response and confers protection from lethal influenza virus infection in mice. J Immunol 2003; 171: 1133-9.
2. Abe T, Hemmi H, Miyamoto H, et al. Involvement of the Toll-like receptor 9 signaling pathway in the induction of innate immunity by baculovirus. J Virol 2005; 79: 2847-58.
3. Abe T, Kaname Y, Wen X, et al. Baculovirus induces type I interferon production through toll-like receptor-dependent and -independent pathways in a cell-type-specific manner. J Virol 2009; 83: 7629-40.
4. Barton G. Viral recognition by Toll-like receptors. Semin Immunol 2007; 19: 33-40.
5. Beck NB, Sidhu JS, Omiecinski CJ. Baculovirus vectors repress phenobarbital-mediated gene induction and stimulate cytokine expression in primary cultures of rat hepatocytes. Gene Ther 2000; 7: 1274-83.
6. Bessis N, GarciaCozar F, Boissier M. Immune responses to gene therapy vectors: influence on vector function and effector mechanisms. Gene Therapy 2004; 1: S10-7.
7. Boulaire J, Zhao Y, Wang S. Gene expression profiling to define host response to baculoviral transduction in the brain. J Neurochem 2009; 109: 1203-14.
8. Burke J. Virus therapy for bladder cancer. Cytokine Growth Factor Rev. 2010 Apr-Jun;21(2-3):99-102.
9. Calcedo R, Vandenberghe LH, Gao G, et al. Worldwide epidemiology of neutralizing antibodies to adeno-associated viruses. J Infect Dis 2009; 199: 381-90.
10. Chen GY, Shiah HC, Su HJ, et al. Baculovirus transduction of mesenchymal stem cells triggers the toll-like receptor 3 pathway. J Virol 2009; 83: 10548-56.
11. Chiong E, Esuvaranathan K. New therapies for non-muscle-invasive bladder cancer. World J Urol. 2010 Feb;28(1):71-8.
12. Cross D, Burmester J. Gene therapy for cancer treatment: past, present and future. Clin Med Res 2006; 4: 218-27.
13. Dumey N, Mongiat-Artus P, Devauchelle P, Lesourd A, Cotard JP, Le Duc A, Marty M, Cussenot O, Cohen-Haguenauer O. In vivo retroviral mediated gene transfer into bladder urothelium results in preferential transduction of tumoral cells. Eur Urol. 2005 Feb;47(2):257-63.
14. Fodor I, Timiryasova T, Denes B, Yoshida J, Ruckle H, Lilly M. Vaccinia virus mediated p53 gene therapy for bladder cancer in an orthotopic murine model. J Urol. 2005 Feb;173(2):604-9.
15. Gomella LG, Mastrangelo MJ, McCue PA, Maguire HC JR, Mulholland SG, Lattime EC. Phase I study of intravesical vaccinia virus as a vector for gene therapy of bladder cancer. J Urol. 2001 Oct;166(4):1291-5.
16. Gronowski AM, Hilbert DM, Sheehan KC, et al. Baculovirus stimulates antiviral effects in mammalian cells. J Virol 1999; 73: 9944-51.
17. Günther JH, Jurczok A, Wulf T, Brandau S, Deinert I, Jocham D, Böhle A. Optimizing syngeneic orthotopic murine bladder cancer (MB49). Cancer Res. 1999 Jun 15;59(12):2834-7.
18. Hacein-Bey-Abina S, Von Kalle C, Schmidt M, et al. LMO2-associated clonal T cell proliferation in two patients after gene therapy for SCID-X1. Science 2003; 302: 415-9.
19. Hadaschik BA, Zhang K, So AI, Fazli L, Jia W, Bell JC, Gleave ME, Rennie PS. Oncolytic vesicular stomatitis viruses are potent agents for intravesical treatment of high-risk bladder cancer. Cancer Res. 2008 Jun 15;68(12):4506-10.
20. Hanel EG, Xiao Z, Wong KK, Lee PW, Britten RA, Moore RB. A novel intravesical therapy for superficial bladder cancer in an orthotopic model: oncolytic reovirus therapy. J Urol. 2004 Nov;172(5 Pt 1):2018-22.
21. Hervas-Stubbs S, Rueda P, Lopez L, et al. Insect baculoviruses strongly potentiate adaptive immune responses by inducing type I IFN. J Immunol 2007; 178: 2361-9.
22. Hofmann C, Sandig V, Jennings G, et al. Efficient gene transfer into human hepatocytes by baculovirus vectors. Proc Natl Acad Sci U S A 1995; 92: 10099-103.
23. Hofmann C, Hüser A, Lehnert W, et al. Protection of baculovirus-vectors against complement-mediated inactivation by recombinant soluble complement receptor type 1. Biol Chem 1999; 380: 393-5.
24. Hu YC. Baculoviral vectors for gene delivery: a review. Curr Gene Ther 2008; 8: 54-65.
25. Hu YC. Baculovirus: a promising vector for gene therapy? Curr Gene Ther. 2010 Jun;10(3):167.
26. Huang X, Yang Y. Innate immune recognition of viruses and viral vectors. Hum Gene Ther 2009; 20: 293-301.
27. Kikuchi E, Menendez S, Ozu C, Ohori M, Cordon-Cardo C, Logg CR, Kasahara N, Bochner BH. Highly efficient gene delivery for bladder cancers by intravesically administered replication-competent retroviral vectors. Clin Cancer Res. 2007 Aug 1;13(15 Pt 1):4511-8.
28. Kikuchi E, Menendez S, Ohori M, Cordon-Cardo C, Kasahara N, Bochner BH. Inhibition of orthotopic human bladder tumor growth by lentiviral gene transfer of endostatin. Clin Cancer Res. 2004 Mar 1;10(5):1835-42.
29. Kimura T, Ohashi T, Kikuchi T, Kiyota H, Eto Y, Ohishi Y. Antitumor immunity against bladder cancer induced by ex vivo expression of CD40 ligand gene using retrovirus vector. Cancer Gene Ther. 2003 Nov;10(11):833-9.
30. Kitajima M, Abe T, Miyano-Kurosaki N, Taniguchi M, Nakayama T, Takaku H. Induction of natural killer cell-dependent antitumor immunity by the Autographa californica multiple nuclear polyhedrosis virus. Mol Ther. 2008 Feb;16(2):261-8.
31. Kitajima M, Takaku H. Induction of antitumor acquired immunity by baculovirus Autographa californica multiple nuclear polyhedrosis virus infection in mice. Clin Vaccine Immunol 2008b; 15: 376-8.
32. Kost TA, Condreay JP, Jarvis DL. Baculovirus as versatile vectors for protein expression in insect and mammalian cells. Nat Biotechnol 2005; 23: 567-75.
33. Kuball J, Wen SF, Leissner J, Atkins D, Meinhardt P, Quijano E, Engler H, Hutchins B, Maneval DC, Grace MJ, Fritz MA, Storkel S, Thüroff JW, Huber C, Schuler M. Successful adenovirus-mediated wild-type p53 gene transfer in patients with bladder cancer by intravesical vector instillation. J Clin Oncol. 2002 Feb 15;20(4):957-65.
34. Lee SS, Eisenlohr LC, McCue PA, Mastrangelo MJ, Lattime EC. Intravesical gene therapy: in vivo gene transfer using recombinant vaccinia virus vectors. Cancer Res. 1994 Jul 1;54(13):3325-8.
35. Loskog AS, Fransson ME, Totterman TT. AdCD40L gene therapy counteracts T regulatory cells and cures aggressive tumors in an orthotopic bladder cancer model. Clin Cancer Res. 2005
36. Malmstrom PU, Loskog AS, Lindqvist CA, Mangsbo SM, Fransson M, Wanders A, Gardmark T, Totterman TH. AdCD40L immunogene therapy for bladder carcinoma--the first phase I/IIa trial. Clin Cancer Res. 2010 Jun 15;16(12):3279-87.
37. Morris BD Jr, Drazan KE, Csete ME, Werthman PE, Van Bree MP, Rosenthal JT, Shaked A. Adenoviral-mediated gene transfer to bladder in vivo. J Urol. 1994 Aug;152(2 Pt 1):506-9.
38. Nayak S, Herzog RW. Progress and prospects: immune responses to viral vectors. Gene Ther 2010; 17: 295-304.
39. Nishibe Y, Kaneko H, Suzuki H, et al. Baculovirus-mediated interferon alleviates dimethylnitrosamine-induced liver cirrhosis symptoms in a murine modelAcMNPV-mediated IFN alleviates LC in a murine model. Gene Ther 2008; 15: 990-7.
40. Pagliaro LC, Keyhani A, Williams D, Woods D, Liu B, Perrotte P, Slaton JW, Merritt JA, Grossman HB, Dinney CP. Repeated intravesical instillations of an adenoviral vector in patients with locally advanced bladder cancer: a phase I study ofp53 gene therapy. J Clin Oncol. 2003 Jun 15;21(12):2247-53.
41. Palmer DH, Young LS, Mautner V. Cancer gene-therapy: clinical trials. Trends Biotechnol 2006; 24: 76-82.
42. Pieroni L, Maione D, La Monica N. In vivo gene transfer in mouse skeletal muscle mediated by baculovirus vectors. Hum Gene Ther 2001; 12: 871-81.
43. Shiau AL, Lin PR, Chang MY, Wu CL. Retrovirus-mediated transfer of prothymosin gene inhibits tumor growth and prolongs survival in murine bladder cancer. Gene Ther. 2001 Nov;8(21):1609-17.
44. Siegel R, Ward E, Brawley O, Jemal A. Cancer statistics, 2011: the impact of eliminating socioeconomic and racial disparities on premature cancer deaths. CA Cancer J Clin. 2011 Jul-Aug;61(4):212-36. Epub 2011 Jun 17.
45. Siemens DR, Crist S, Austin JC, Tartaglia J, Ratliff TL Comparison of viral vectors: gene transfer efficiency and tissue specificity in a bladder cancer model.. J Urol. 2003 Sep;170(3):979-84.
46. Strauss R, Hüser A, Ni S, Tuve S, Kiviat N, Sow PS, Hofmann C, Lieber A. Baculovirus-based vaccination vectors allow for efficient induction of immune responses against plasmodium falciparum circumsporozoite protein. Mol Ther. 2007 Jan;15(1):193-202.
47. Sutton MA, Berkman SA, Chen SH, Block A, Dang TD, Kattan MW, Wheeler TM, Rowley DR, Woo SL, Lerner SP. Adenovirus-mediated suicide gene therapy for experimental bladder cancer. Urology. 1997 Feb;49(2):173-80.
48. Suzuki T, Chang MO, Kitajima M, Takaku H. Baculovirus activates murine dendritic cells and induces non-specific NK cell and T cell immune responses. Cell Immunol. 2010;262(1):35-43.
49. Wang S, Balasundaram G. Potential cancer gene therapy by baculoviral transduction. Curr Gene Ther. 2010 Jun;10(3):214-25
50. Zlotta AR, Fleshner NE, Jewett MA. The management of BCG failure in non-muscle-invasive bladder cancer: an update. Can Urol Assoc J. 2009 Dec;3(6 Suppl 4):S199-205.

## Claims

1. A baculoviral vector for use in stimulating an anti-tumor immune response against a bladder cancer cell in a subject in need of such treatment, the baculoviral vector either (i) having no transgene or (ii) having only a therapeutic transgene operably linked to a promoter that drives expression of the therapeutic transgene in a bladder cell of the subject to increase or supplement the anti-tumor response, wherein the use comprises intravesical instillation of the baculoviral vector into the bladder of the subject.

2. The baculoviral vector for use according to claim 1, the use comprising reducing or preventing recurrence of bladder cancer, wherein the subject has previously had bladder cancer.

3. The baculoviral vector for use according to claim 1 or claim 2, wherein the baculoviral vector does not contain any transgene.

4. The baculoviral vector for use according to claim 1 or claim 2, wherein the baculoviral vector has only a therapeutic transgene operably linked to a promoter that drives expression of the therapeutic transgene in a bladder cell of the subject to increase or supplement the anti-tumor response.

5. The baculoviral vector for use according to claim 4, wherein the transgene is CD40L or IL-15.

6. The baculoviral vector for use according to claim 4 or 5, wherein the promoter comprises the human cytomegalovirus immediate early promoter, preferably the sequence set forth in SEQ ID NO: 1.

7. The baculoviral vector for use according to any one of claims 1 to 6, wherein the baculoviral vector further comprises post-transcriptional regulatory elements from the woodchuck hepatitis virus, in the 3' untranslated region of the transgene, preferably the sequence set forth in SEQ ID NO: 2.

8. The baculoviral vector for use according to any one of claims 1 to 7, wherein the baculoviral vector further comprises the R segment and at least a portion of the U5 sequence of the long terminal repeat from the human T-cell leukemia virus type 1, in the 5' untranslated region of the transgene, preferably the sequence set forth in SEQ ID NO: 3.

9. The baculoviral vector for use according to any one of claims 1 to 8, wherein the use does not involve use of a transfection agent.

10. The baculoviral vector for use according to any one of claims 1 to 8, further comprising adding a transfection agent either prior to or concurrently with said instillation.

11. The baculoviral vector for use according to claim 10, wherein the transfection agent is poly-L-lysine, sodium oxychlorosene, dodecyl-B-dd-maltoside (DDM), or sodium dodecyl sulfate (SDS), or any combination thereof.

## Patentansprüche

1. Baculovirusvektor zur Verwendung bei der Stimulierung einer Antitumorimmunantwort gegen eine Blasenkrebszelle in einem Subjekt, das einer solchen Behandlung bedarf, wobei der Baculovirusvektor entweder (i) kein Transgen umfasst oder (ii) nur ein therapeutisches Transgen umfasst, das funktionsfähig mit einem Promotor verbunden ist, der die Expression des therapeutischen Transgens in einer Blasenzelle des Subjekts antreibt, um die Antitumorantwort zu erhöhen oder zu ergänzen, wobei die Verwendung die intravesikale Instillation des Baculovirusvektors in die Blase des Subjekts umfasst.

2. Baculovirusvektor zur Verwendung nach Anspruch 1, wobei die Verwendung das Reduzieren oder Verhindern des Wiederauftretens von Blasenkrebs umfasst, wobei das Subjekt zuvor Blasenkrebs hatte.

3. Baculovirusvektor zur Verwendung nach Anspruch 1 oder Anspruch 2, wobei der Baculovirusvektor kein Transgen enthält.

4. Baculovirusvektor zur Verwendung nach Anspruch 1 oder Anspruch 2, wobei der Baculovirusvektor nur ein therapeutisches Transgen aufweist, das funktionell mit einem Promotor verbunden ist, der die Expression des therapeutischen Transgens in einer Blasenzelle des Subjekts antreibt, um die Antitumorantwort zu erhöhen oder zu ergänzen.

5. Baculovirusvektor zur Verwendung nach Anspruch 4, wobei das Transgen CD40L oder IL-15 ist.

6. Baculovirusvektor zur Verwendung nach Anspruch 4 oder 5, wobei der Promotor den Immediate-Early-Promotor des humanen Cytomegalovirus umfasst, vorzugsweise die in SEQ ID NO: 1 angegebene Sequenz.

7. Baculovirusvektor zur Verwendung nach einem der Ansprüche 1 bis 6, wobei der Baculovirusvektor ferner posttranskriptionale regulatorische Elemente aus dem Murmeltier-Hepatitisvirus in der 3'-untranslatierten Region des Transgens umfasst, vorzugsweise die in SEQ ID NO: 2 angegebene Sequenz.

8. Baculovirusvektor zur Verwendung nach einem der Ansprüche 1 bis 7, wobei der Baculovirusvektor ferner das R-Segment und mindestens einen Abschnitt der U5-Sequenz des langen terminalen Repeats aus dem humanen T-Zell-Leukämievirus Typ 1 in der 5'-untranslatierten Region des Transgens umfasst, vorzugsweise die in SEQ ID NO: 3 angegebene Sequenz.

9. Baculovirusvektor zur Verwendung nach einem der Ansprüche 1 bis 8, wobei die Verwendung nicht die Verwendung eines Transfektionsmittels beinhaltet.

10. Baculovirusvektor zur Verwendung nach einem der Ansprüche 1 bis 8, ferner umfassend die Zugabe eines Transfektionsmittels entweder vor oder gleichzeitig mit der Instillation.

11. Baculovirusvektor zur Verwendung nach Anspruch 10, wobei das Transfektionsmittel Poly-L-Lysin, Natriumoxychlorosin, Dodecyl-B-ddmaltosid (DDM) oder Natriumdodecylsulfat (SDS) oder eine beliebige Kombination davon ist.

## Revendications

1. Vecteur baculoviral pour une utilisation au niveau de la stimulation d'une réponse immunitaire anti-tumorale à l'encontre d'une cellule du cancer de la vessie chez un sujet qui a besoin d'un tel traitement, le vecteur baculoviral soit (i) ne comportant pas de transgène, soit (ii) comportant seulement un transgène thérapeutique qui est lié de façon fonctionnelle à un promoteur qui pilote l'expression du transgène thérapeutique dans une cellule de la vessie du sujet de manière à augmenter ou compléter la réponse anti-tumorale, dans lequel l'utilisation comprend une instillation intravésicale du vecteur baculoviral à l'intérieur de la vessie du sujet.

2. Vecteur baculoviral pour une utilisation selon la revendication 1, l'utilisation comprenant la réduction ou la prévention de la récidive du cancer de la vessie, dans lequel le sujet a eu précédemment un cancer de la vessie.

3. Vecteur baculoviral pour une utilisation selon la revendication 1 ou la revendication 2, dans lequel le vecteur baculoviral ne contient pas de transgène.

4. Vecteur baculoviral pour une utilisation selon la revendication 1 ou la revendication 2, dans lequel le vecteur baculoviral comporte seulement un transgène thérapeutique lié de façon fonctionnelle à un promoteur qui pilote une expression du transgène thérapeutique dans une cellule de la vessie du sujet de manière à augmenter ou compléter la réponse anti-tumorale.

5. Vecteur baculoviral pour une utilisation selon la revendication 4, dans lequel le transgène est CD40L ou IL-15.

6. Vecteur baculoviral pour une utilisation selon la revendication 4 ou la revendication 5, dans lequel le promoteur comprend le promoteur précoce immédiat du cytomégalovirus humain, de préférence la séquence décrite selon SEQ ID NO : 1.

7. Vecteur baculoviral pour une utilisation selon l'une quelconque des revendications 1 à 6, dans lequel le vecteur baculoviral comprend en outre des éléments régulateurs post-transcriptionnels qui proviennent du virus de l'hépatite de la marmotte, dans la région non traduite 3' du transgène, de préférence la séquence décrite selon SEQ ID NO : 2.

8. Vecteur baculoviral pour une utilisation selon l'une quelconque des revendications 1 à 7, dans lequel le vecteur baculoviral comprend en outre le segment R et au moins une partie de la séquence U5 de la répétition terminale longue qui provient du type 1 du virus de la leucémie à cellules T humaine, dans la région non traduite 5' du transgène, de préférence la séquence décrite selon SEQ ID NO : 3.

9. Vecteur baculoviral pour une utilisation selon l'une quelconque des revendications 1 à 8, dans lequel l'utilisation ne met pas en jeu l'utilisation d'un agent de transfection.

10. Vecteur baculoviral pour une utilisation selon l'une quelconque des revendications 1 à 8, comprenant en outre l'ajout d'un agent de transfection soit avant ladite instillation, soit concurremment à celle-ci.

11. Vecteur baculoviral pour une utilisation selon la revendication 10, dans lequel l'agent de transfection est la poly-L-lysine, l'oxychlorosène de sodium, le dodécyl-B-dd-maltoside (DDM) ou le dodécylsulfate de sodium (SDS) ou une quelconque combinaison afférente.
